(19)

**Europäisches Patentamt**
**European Patent Office**
**Office européen des brevets**

(11) **EP 4 670 703 A1**

(12) **EUROPEAN PATENT APPLICATION**

(43) Date of publication:
**31.12.2025   Bulletin 2026/01**

(21) Application number: **24382681.5**

(22) Date of filing: **24.06.2024**

(51) International Patent Classification (IPC):
*A61K 6/20* (2020.01)          *A61K 8/81* (2006.01)
*C08F 2/28* (2006.01)          *C11D 3/48* (2006.01)

(52) Cooperative Patent Classification (CPC):
**A61K 6/20; A61K 8/8152; C08F 2/28**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Ivoclar Vivadent AG**
  **9494 Schaan (LI)**
- **Universidad del País Vasco/Euskal Herriko Unibertsitatea**
  **48940 Leoia, Bikaia (ES)**

(72) Inventors:
- **Schnur,Thomas**
  **9488 Schellenberg (LI)**

- **Lendenmann, Urs**
  **9472 Grabs (CH)**
- **Angermann, Jörg**
  **7320 Sargans (CH)**
- **Moszner, Norbert**
  **99441 Grossschwabhausen/OT Hohlstedt (DE)**
- **Catel, Yohann**
  **9475 Sevelen (CH)**
- **Bolis, Carlo**
  **7206 Igis (CH)**
- **Asua, José**
  **20018 Donostia/San Sebastian (ES)**
- **Kuhnt, Todias**
  **20015 Donostia/San Sebastian (ES)**

(74) Representative: **Uexküll & Stolberg**
**Partnerschaft von**
**Patent- und Rechtsanwälten mbB**
**Beselerstraße 4**
**22607 Hamburg (DE)**

(54) **DENTAL VARNISHES BASED ON CATIONIC POLYMER DISPERSIONS**

(57)   A cationic polymer dispersion comprising at least one copolymer, water, at least one surfactant, and 0.3 to 3.0 wt.% of at least one organic and/or inorganic fluoride source, which is essentially free from low-boiling organic solvents. The dispersion is suitable as a varnish or coating for the fluoridation of teeth and for the treatment of prophylaxis of caries.

**EP 4 670 703 A1**

## Description

## TECHNICAL FIELD

The present invention relates to copolymer compositions, which are particularly suitable as dental materials for the production of dental varnishes, lacquers or coatings. Specifically, the invention is directed towards materials for use in the therapy of caries showing a fluoride release.

## BACKGROUND

Dental fluoride varnishes are liquid fluoride-containing compositions, which are applied to the tooth's surface usually by the dentists, dental hygienists or other health care professionals (E. D. Beltrán-Aguilar, J. W. Goldstein, S. A. Lockwood, J. Amer. Dent. Ass. 131 (2000), 589-596).

The main components of most varnishes are similar. They are composed of resin, such as colophony or polyurethane-based resin, alcohol, and a fluoride source, e.g., sodium fluoride or difluorosilane. Alcohols, such as ethanol or others, are utilized as solvents to keep the varnish in a liquid form and facilitate its application. When the varnish comes into contact with air, the solvents evaporate and the vanish becomes adherent to the tooth surfaces, thereby increasing the duration of fluoride exposure. They can stay attached to the surface for several hours to a few days after application, but are not intended to remain permanently on the teeth. Fluoride varnishes were found to be effective for preventing occlusal caries in children with a moderate to high risk of caries (A. Baik, N. Alamoudi, A. El-Housseiny, A. Altuwirqi, Dent. J. 9 (2021), 64, 1-15).

DE 102 42 476 A1 discloses compositions comprising one or more poorly water-soluble antibiotic salts, optionally a readily water-soluble antibiotic and one or more excipients, suspended in a homogeneous polymer blend, consisting of one or more hydrophobic non-ionic polymers selected from PVC, chlorinated PVC, poly(vinylidene chloride), poly(vinyl fluoride), poly(vinylidene fluoride), the copolymers of vinyl chloride with one or more non-ionic monomers, and one or more hydrophilic polymers selected from the group consisting of polyethers. The compositions are said to be suitable for coating, e.g., plastic tubing and plastic bodies suitable as medical implants, and to release the antibiotics in an aqueous environment over a period of days.

DE 10 2008 010 464 A1 discloses a dental composition comprising the reaction product of a polyisocyanate, a perfluorinated alcohol, an ethylenically unsaturated alcohol, such as a hydroxy(meth)acrylate, and optionally a reactive diluent. The materials are said to be useful for coating teeth and to provide the tooth surfaces with a hydrophobic/oleophobic coating that protects the tooth surfaces from plaque formation.

WO 2010/043606 A1 discloses a coating composition comprising an organic microbicidal compound, such as chlorhexidine, and a condensate of at least one silicon compound which can be hydrolyzed and which comprises at least one organic group which cannot be hydrolyzed. The composition can be used to produce silver-free microbicidal coatings that are said to have a high microbicidal activity and largely prevent the formation of biofilms. To achieve anti-adhesive properties, the compositions may comprise fluorosilanes.

EP 2 705 825 A1 discloses a fluoride-containing varnish for application to the tooth surface, which contains 25 to 87.5 wt.% organic solvent, 2 to 50 wt.% water, 5 to 50 wt.% water-insoluble film-forming agent, 0.5 to 10 wt.% of a dissolved inorganic fluoride source and optionally 5 to 25 wt.% plasticizer. The varnish is particularly suitable for use in the treatment of hypersensitive teeth and/or tooth necks, in the prophylaxis of caries, in the treatment of incipient caries lesions, the inhibition of demineralization and/or tooth erosion. By the combined use of organic solvents and water, good film-building properties and fluoride release are achieved.

WO 2015/084315 A1 discloses an oral care composition, comprising from 30 to 90 wt.% of a silsesquioxane silicone resin and from 10 to 50 wt.% of a solvent, e.g., ethanol. The composition may further comprise an oral care active agent selected from one or more of a fluoride ion source, a desensitizing agent, a tooth whitening agent, a tooth bleaching agent, an antibacterial agent, an anti-calculus agent and mixture thereof. The composition preferably comprises less than 3 wt.% water. It was allegedly found that combining a solvent with a silsesquioxane silicone resin results in an oral care composition which can provide a film that results in a visibly shinier enamel surface.

DE-OS 10 2007 040 569 A1 discloses varnishes for the prophylaxis against caries and for the protection of teeth and gums, based on a photopolymerizable composition comprising 10 to 70 wt.% of photopolymerizable monomers, 29.5 to 70 wt.% of a water miscible organic solvent, 0.4 to 3 wt.% of photoinitiators and activators, and 0.1 to 3.0 wt.% of an antibiotic agent, such as chlorhexidine, alexidine, polyhexanide, or amine fluoride.

The above dental varnishes comprise organic solvents, mostly ethanol. Organic solvents often have a strong and unpleasant odor or taste. Moreover, they can cause a burning sensation, which is particularly unfavorable for treating children. On the other hand, dental varnishes should be insoluble in water to prevent premature dissolution by saliva and should quickly dry under oral conditions, which is why they usually contain organic solvents. However, low water solubility is often associated with low water tolerance, which can impair the formation of a cohesive film. This is because it is difficult to keep the teeth completely dry during the intraoral application of a varnish and water residues on the teeth can cause the precipitation of varnish components. In addition, the known fluoride varnishes tend to pull strings when applied.

The known aqueous fluoride varnishes based on acrylic resins contain maximally 0.15 wt.% fluoride, because the dispersions gel or precipitate at higher fluoride concentrations, and dental varnishes based on aqueous cationic polymer dispersions with a fluoride content of more than 0.2 wt.% are not known.

US 2008/0194753 A1 discloses aqueous dispersions of water-soluble and/or water-swellable anionic polymers which are obtainable by free-radical polymerization of ethylenically unsaturated, anionic monomers, e.g., acrylic or methacrylic acid, crotonic acid, maleic acid, fumaric acid, vinylsulfonic acid, styrenesulfonic acid or vinylphosphonic acid, in an aqueous medium in the presence of at least one stabilizer selected from water-soluble polymers. It is an object of this application to avoid the use of stabilizing inorganic salts in the preparation of the dispersions. The compositions are said to be suitable as thickeners for paper coating slips, pigment print pastes, dental compounds, agrochemical agents etc. Dental varnishes are not disclosed.

CN 110591008 A discloses a process for the preparation of cationic alkyl core-shell emulsion polymers. In this process, cationic strongly hydrophilic monomers, such as N-octadecyl acrylamide or octadecyl vinyl ether, are reacted with long-alkyl chain hydrophobic monomers, such as cyclopentyl acrylate or cyclohexyl methacrylate, in the presence of an emulsifier, such as tetramethylammonium fluoride or tetramethylammonium chloride, an initiator, e.g., dilauroyl peroxide or tert-Bu peroxypivalate, and a chain transfer agent, such as sodium hypophosphite or sodium methallyl sulfonate. The polymers are said to be suitable for coating the surface of concrete buildings with a waterproof coating having good mechanical properties.

EP 0 286 009 A2 discloses cationic polymer dispersions which comprise biocidal cationic surface active quaternary organic ammonium compounds. They are said to be useful as fungicidal, bactericidal or algicidal treatment agents for the preservation of wood, for emulsion paint films, for polymer plasters and for synthetic resin plasters. The polymer dispersions can be obtained by emulsion polymerization, for example by azodinitrile-initiated aqueous polymerization of butyl acrylate, methyl methacrylate, and 2-hydroxyethyl methacrylate in the presence of 1-dodecylpyridinium chloride.

CN 110627963 A discloses a process for the preparation of quaternary ammonium salt and quaternary phosphine salt cationic core-shell emulsion polymers. In this process, cationic strongly hydrophilic monomers are reacted with small hydrophobic monomers comprising a benzene ring by emulsion polymerization. The polymers are said to be suitable for coating cement surfaces with a waterproof coating that is more environmentally friendly than oil-based coatings, adheres more strongly to the surface than anionic coatings, and has a better and more durable waterproof effect.

## SUMMARY

It is an object of the present invention to provide dental vanishes which do not have the above disadvantages. They should be easy to handle and have a neutral or pleasant odor and taste. In addition, they should have low toxicity and form cohesive, smooth, colorless, water-tolerant, and non-erosive films that adhere well to the tooth surface (enamel and dentin). Furthermore, the compositions should not pull strings and should not produce a burning sensation when applied.

It is a further object of the invention to provide dental compositions that can deliver active ingredients such as antimicrobial compounds, compounds having a desensitizing effect, bleaching agents and, especially, fluorides to the teeth. It is a particular object of the invention to provide dental varnishes for professional use that comprise more than 0.2 wt.% fluoride.

## DETAILED DESCRIPTION

According to the invention, these objects are achieved by cationic polymer dispersions comprising:

(a) 5 to 41 wt.%, preferably 7 to 35 wt.%, and more preferably 15 to 30 wt.% of at least one copolymer,
(b) 50 to 94 wt.%, preferably 60 to 91 wt.%, and more preferably 65 to 83 wt.% of water,
(c) 0.5 to 6.0 wt.%, preferably 0.6 to 5.5 wt.%, and more preferably 0.7 to 5.0 wt.% of at least one surfactant and
(d) 0.3 to 3.0 wt.%, preferably 0.5 to 2.5 wt.% and more preferably 1.0 to 2.0 wt.% of at least one organic and/or inorganic fluoride source (calculated as $F^-$).

Unless stated otherwise, all weight percentages herein refer to the total weight of the dispersion.

The cationic polymer dispersions of the invention contain essentially no low boiling organic solvent. The wording that the dispersions contain essentially no low-boiling solvents is to be understood as meaning that the content of such solvents is less than 5 wt. %, preferably less than 2 wt.% and more preferably less than 1 wt.%.

According to the invention, low-boiling organic solvents are organic solvents having a boiling point of less than 100°C, preferably less than 160°C, more preferably less than 180°C and most preferably less than 205°C. The boiling point is determined at atmospheric pressure. In particular, the cationic polymer dispersions of the present invention do not comprise methylene chloride, methyl t-butyl ether, acetone, 1,2-dimethoxy-ethane, methanol, ethanol, ethyl acetate, 1-propanol, 2-propanol, 2-butanone, t-butanol and 2-butanol, more preferably also no dimethylformamide and 1,4-dioxane, and most preferably also no N-methyl-2-pyrrolidone.

The at least one copolymer (a) is preferably a copolymer which is obtainable by free-radical copolymerization of a monomer mixture comprising:

(a1) 5 to 70 mol%, preferably 10 to 65 mol% and more preferably 15 to 60 mol% of at one least rigid (meth)acrylate or vinyl monomer and
(a2) 30 to 95 mol%, preferably 35 to 90 mol%, and more preferably 40 to 85 mol% of at least one flexible (meth)acrylate or vinyl monomer.

Unless stated otherwise, all mole percentages herein refer to the total molar amount of all monomers.

The **monomer (a1)** is a rigid (meth)acrylate or vinyl monomer or a mixture thereof. According to the invention, rigid monomers are monomers that form homopolymers with a glass transition temperature ($T_G$) of from 30°C to 130°C and preferably from 50°C to 120°C. Particularly preferred are rigid monomers with a water solubility of less than 0.1 g/L at room temperature (22°C).

Preferred rigid (meth)acrylate monomers are methyl methacrylate, phenyl, naphthyl, benzyl, adamantyl (meth)acrylate or isobornyl (meth)acrylate, 2-[(methoxycarbonyl)-amino]ethyl (meth)acrylate, 2-[(propoxycarbonyl)amino]ethyl (meth)acrylate, 2-[(isopropoxycarbonyl)amino]ethyl (meth)acrylate, 2-[(butoxycarbonyl)amino]ethyl (meth)-acrylate, 2-[(hexyloxycarbonyl)amino]ethyl (meth)acrylate, 2-[(cyclohexyloxycarbonyl)amino]ethyl (meth)acrylate, 2-[(benzyloxycarbonyl)amino]ethyl (meth)acrylate, 2-[(ethylcarbamoyl)oxy]ethyl (meth)acrylate, 2-[(propylcarbamoyl)oxy]ethyl (meth)acrylate, 2-[(isopropylcarbamoyl)oxy]ethyl (meth)acrylate, 2-[(hexylcarbamoyl)oxy]ethyl (meth)acrylate, 2-[(benzylcarbamoyl)oxy]ethyl (meth)acrylate, 2-[(2-tetrahydrofurfuryloxycarbonyl)amino]ethyl (meth)acrylate, 2-[(2-oxo-1,3-dioxolan-4-yl)methoxycarbonylamino]ethyl (meth)acrylate, (meth)acrylic acid 2-(furan-2-yl-methoxycarbonylamino)ethyl ester, (1,3-dioxolan-2-on-4-yl) methyl (meth)acrylate, 2-phenoxyethyl (meth)acrylate, 2-(o-biphenyloxy)ethyl (meth)acrylate, 2-hydroxy-3-phenoxypropyl (meth)acrylate, 2-phenoxypropyl (meth)acrylate, 2-(p-cumylphenoxy)-ethyl (meth)acrylate. Tricyclodecane (meth)acrylate, tricyclodecanemethyl (meth)-acrylate and 1,7,7-trimethylbicyclo[2.2.1]heptan-2-yl (meth)acrylate. Methyl and benzyl methacrylate are the most preferred (meth)acrylate monomers.

Preferred rigid vinyl monomers are styrene and styrene derivatives, such as p-methylstyrene, p-ethylstyrene, p-methoxystyrene, α-methyl styrene, divinyl benzene and acrylonitrile.

The most preferred rigid monomers are methyl methacrylate and styrene.

The monomer mixture comprises 5 to 70 mol%, preferably 10 to 65 mol% and more preferably 15 to 60 mol% of at least one rigid (meth)acrylate or vinyl monomer or a mixture thereof.

The **monomer (a2)** is a flexible (meth)acrylate or vinyl monomer or a mixture thereof. According to the invention, flexible monomers are monomers that form homopolymers with a glass transition temperature ($T_G$) of from -70°C to 20°C, preferably from -65°C to 0°C, and most preferably from -60 °C to -20 °C. Preferred are flexible monomers with a water solubility of less than 0.1 g/L at room temperature (22°C).

Preferred flexible (meth)acrylate monomers are alkyl or cycloalkyl (meth)acrylates, such as n-butyl methacrylate ($T_G$: 20°C), n-pentyl methacrylate ($T_G$: 10°C), n-hexyl methacrylate ($T_G$: -5°C), 2-ethylhexyl methacrylate ($T_G$: -10°C), n-octyl methacrylate ($T_G$: -20°C), n-dodecyl methacrylate ($T_G$: -65°C), tetradecyl methacrylate ($T_G$: -9°C) or hexadecyl methacrylate ($T_G$: 16°C) as well as the corresponding acrylates, and hydroxyalkyl (meth)acrylates such as 2-hydroxyethyl acrylate ($T_G$: -14°C), 2-octyl acrylate ($T_G$: -44°C) and 2-hydroxypropyl acrylate.

Preferred flexible vinyl monomers are vinyl acetate, and the vinyl esters of saturated monocarboxylic acids with a highly branched structure and 5 to 12, preferably 9 or 10, carbon atoms. Particularly preferred are the vinyl esters of neodecanoic acid and neononanoic acid, i.e., vinyl neodecanoate and vinyl neononanoate.

The most preferred flexible monomers are n-butyl acrylate ($T_G$: -54°C), n-hexyl acrylate ($T_G$: -57°C) and 2-ethylhexyl acrylate ($T_G$: -50°C).

The monomer mixture comprises 30 to 95 mol%, preferably 35 to 90 mol% and more preferably 40 to 85 mol% of at least one flexible monomer, most preferably of at least one (meth)acrylate monomer.

According to the invention, glass-transition temperatures ($T_G$) of homopolymers and copolymers are measured by differential scanning calorimetry, preferably with a TA Instruments Differential Scanning Calorimeter DSC Q2000 (TA Instruments, New Castle), in modulated mode at a heating rate of 10°C min$^{-1}$ with an amplitude of 2°C and a period of 60 s. Samples are cooled below the $T_G$s of the phases, e.g., -50°C, and two thermal scans are performed up to 150°C and the average value is calculated.

The monomer mixture can additionally comprise one or more additional radically polymerizable monomers that are selected from basic nitrogen-containing monomers, zwitterionic monomers, antimicrobial monomers, preferably cationic monomers and/or adhesive monomers, and mixtures thereof. According to the invention, basic nitrogen-containing monomers are monomers comprising one or more primary, secondary and/or tertiary amino groups.

Basic nitrogen-containing monomers can be used to introduce cationic groups into the copolymers by subsequent addition of an acid leading to protonation of the amino group. Preferred nitrogen-containing monomers are (meth)acrylates, in particular N,N-dialkylaminoalkyl (meth)acrylates, such as 2-(dimethylamino)ethyl (meth)acrylate, 2-(diethylamino)ethyl (meth)acrylate, 3-(dimethylamino)propyl (meth)acrylate, and 3-(diethylamino)propyl (meth)acrylate, as well as N,N-dialkylaminoalkyl (meth)acrylamides, such as dialkylaminoethyl (meth)acrylamides or dialkylaminopropyl (meth)acrylamides. Particularly preferred are 2-(dimethylamino)ethyl methacrylamide and 2-(dimethylamino)ethyl acrylamide.

The monomer mixture may comprise 0 to 15 mol%, preferably 0.1 to 15 mol%, more preferably 0.1 to 10 mol% and most preferably 0.1 to 5 mol% of one or more basic nitrogen-containing monomers.

Preferred cationic monomers are (meth)acryloyloxyalkyltrialkylammonium halides, such as [2-(methacryloyloxy)ethyl]trimethylammonium chloride, [2-(acryloyloxy)-ethyl]trimethylammonium chloride, [2-(methacryloyloxy)ethyl]trimethylammonium bromide, [2-(acryloyloxy)ethyl]trimethylammonium bromide, [3-(methacryloyloxy)-propyl]trimethylammonium chloride, [4-(methacryloyloxy)butyl]trimethylammonium chloride, [2-(methacryloyloxy)ethyl]triethylammonium chloride and [2-(acryloyloxy)-ethyl]triethylammonium chloride.

Furthermore, polymerizable quaternary ammonium salts are preferred as cationic monomers, in particular (3-methacryloyloxypropyl)dimethyldodecylammonium bromide, N-(2-methacryloyloxyethyl)-N,N-dimethyl cetylammonium chloride, dimethyl(4-vinylbenzyl)hexadecylammonium chloride, dimethyl-(4-vinylbenzyl)dodecylammonium chloride and 2-dimethyl-2-dodecyl-1-methacryloxyethylammonium iodine. Pyridinium monomers such as 12-(methacryloyloxy)dodecyl)pyridinium bromide, 11-(methacryloyloxy)undecyl)pyridinium bromide, 10-(methacryloyloxy)-decylpyridinium bromide, 8-(methacryloyloxy)octylpyridinium bromide, 6-(methacryl-oyloxy)hexyl)pyridinium bromide, 4-(methacryloyloxy)-2,2-dimethylpropyl)pyridinium bromide are also preferred.

Further preferred cationic monomers are N-4-(2-methacryloyloxyethyl)-phenyl-N'-4-chlorphenylbiguanide hydrochloride, 1-vinyl-3-octylimidazolium tetrafluoro borate, 1-vinyl-3-butyl-imidazolium hexafluorophosphate, 1-vinyl-3-hexy-limidazolium hexafluorophosphate, 1-vinyl-3-octyl-imidazolium hexafluorophosphate and imidazolium compounds, such as 1-methylacryoyloxyethyl-3-butyl-imidazolium tetrafluoro borate, 1-(2-(((2-(methacryloyloxy)ethyl)carbamoyl)oxy)ethyl)-3-decyl-imidazolium bromide, 1-(2-(((2-(methacryloyloxy)ethyl)carbamoyl)oxy)ethyl)-3-dodecyl-imidazolium bromide or 1-(2-(((2-(methacryloyloxy)ethyl)carbamoyl)oxy)ethyl)-3-decyl-imidazolium bromide.

Particularly preferred cationic monomers are [2-(methacryloyloxy)ethyl]trimethylammonium chloride, [2-(methacryloyloxy)ethyl]-triethylammonium chloride, methacryl-oyloxydodecylpyridinium bromide, methacryloxyethylhexadecylmethylammonium bromide, 12-(methacryloyloxy)dodecyl)pyridinium bromide, 11-(methacryloyloxy)undecyl)pyridinium bromide, and 10-(methacryloyloxy)decylpyridinium bromide.

The monomer mixture may comprise 0 to 15 mol%, preferably 0.1 to 15 mol%, more preferably 0.1 to 10 mol%, and most preferably 1 to 5 mol% of one or more cationic monomers.

Zwitterionic monomers comprise both cationic and anionic groups in the same molecule. Preferred zwitterionic monomers are sulfobetaine monomers, e.g., 3-{2-(methacryloyloxy)ethyl]dimethylammonio} propane-1-sulfonate, 4-[2-methacryloyl-oxy)ethyl]dimethylammonio]butane-1-sulfonate, 4-[[2-acryloyloxy)ethyl]dimethylam-monio]butane-1-sulfonate, 3-[bis[methacryloyloxy)ethyl](methylammonio]propane-1-sulfonate, 3-[(3-methacrylamidopropyl)dimethylammonio]propane-1-sulfonate, 4-[(3-methacrylamidopropyl)dimethylammonio]butane-1-sulfonate and 3-[(3-acrylamidopropyl)dimethylammonio]propane-1-sulfonate.

Furthermore, carboxybetaine monomers, such as 2-[[2-(methacryloyloxy)ethyl]-dimethylammonio] acetate, 3-[[2-(methacryloyloxy)ethyl]dimethylammonio]propionate or 3-[(3-acrylamidopropyl)dimethylammonio]propionate, and phosphobetaine monomers, such as 2-methacryloyloxyethyl phosphorylcholine and 2-acryloyloxyethyl phosphorylcholine, are preferred as zwitterionic monomers.

A particularly preferred zwitterionic monomer is 3-[[2-(methacryloyloxy)ethyl]dimethylammonio]propane-1-sulfonate.

The monomer mixture may comprise 0 to 15 mol%, preferably 0 to 10 mol%, and more preferably 0 to 5 mol% of one or more zwitterionic monomers.

Preferred antimicrobial monomers are nonionic monomers such as 2-(meth)acryloyloxyethyl p-hydroxybenzoate, eugenyl (meth)acrylate (4-allyl-2-methoxyphenyl (meth)acrylate), 2-(4-allyl-2-methoxyphenoxy)-1-hydroxyethyl (meth)acrylate, thymol (meth)acrylate (2-isopropyl-5-methylphenyl (meth)acrylate), 2-(((thiazol-5-ylmethoxy)carbonyl)amino)ethyl (meth)acrylate, (((2-(benzo[d]thiazol-2-ylthio)-ethoxy)carbonyl)amino)methyl (meth)acrylate and 2-((3,4-dichloro-5-oxo-2,5-dihydrofuran-2-yl)oxy)ethyl (meth)acrylate. Further preferred antimicrobial monomers are cationic monomers and in particular cationic monomers with quaternary ammonium groups such as the polymerizable quaternary ammonium salts defined above.

The monomer mixture may comprise 0 to 10 mol%, preferably 0 to 8 mol%, and more preferably 0 to 5 mol% of one or more nonionic antibacterial monomers.

When applied to teeth, the cationic copolymer dispersions of the present invention form smooth films that adhere well to dental enamel. If desired, adhesion can be improved by using adhesive monomers to prepare the copolymers. Adhesive monomers are monomers which can interact with the hard tissue of the tooth (enamel and dentin) to form ionic or coordinative bonds. For instance, monomers comprising one or more acidic or chelating groups can react with calcium ions. Thus, adhesive monomers can improve the adhesion on enamel.

Preferred adhesive monomers are radically polymerizable monomers with at least one acid group, preferably a carboxylic acid group, a phosphoric acid ester group and/or a phosphonic acid group, more preferably a carboxylic acid group or phosphoric acid ester group, and most preferably a monohydrogen phosphate or dihydrogen phosphate group. Adhesive monomers are also referred to herein as acidic monomers. Preferred radically polymerizable groups are acrylate groups and in particular methacrylate groups. Thus, monomers comprising at least one (meth)acrylate group and at least one carboxylic acid group, phosphonic acid group or phosphoric acid ester group are quite particularly preferred. Polymerizable carboxylic acids and dihydrogen phosphates are most preferred.

Preferred acidic monomers containing a COOH group are (meth)acrylic acid, 4-(meth)acryloyloxyethyl trimellitic acid, 10-methacryloyloxydecyl malonic acid, N-(2-hydroxy-3-methacryloyloxypropyl)-N-phenyl glycine, and 2-carboxyethyl acrylate (CEA).

Preferred radically polymerizable phosphonic acids are 2-[4-(dihydroxyphosphoryl)-2-oxa-butyl]acrylic acid and its esters, such as ethyl 2-[4-(dihydroxyphosphoryl)-2-oxa-butyl]acrylate (EDOBA) and 2,4,6-trimethylphenyl 2-[4-(dihydroxyphosphoryl)-2-oxa-butyl]acrylate. EDOBA is most preferred.

Preferred radically polymerizable dihydrogen phosphates are 2-methacryloyloxyethylphenyl hydrogen phosphate, 10-methacryloyloxydecyl dihydrogen phosphate (MDP), glycerine dimethacrylate dihydrogen phosphate and dipentaerythritol pentamethacryloyloxy dihydrogen phosphate. MDP is mostly preferred.

The monomer mixture may comprise 0 to 10 mol%, preferably 0 to 7 mol%, more preferably 0.1 to 5 mol%, and most preferably 1 to 5 mol% of one or more acidic monomers.

Preferred chelating monomers are monomers that contain one or more chelating groups, which can interact with calcium ions. Preferred examples are ß-ketones, ß-keto esters, and monomers with chelating alkylenediamine di-, tri- or tetraacetic groups. A preferred chelating monomer is the commercially available ß-keto ester 2-acetoacetoxyethyl methacrylate. Suitable styryl group containing polymerizable ß-diketones are described in CN 102079693 B. Further examples of chelating monomers are the reaction products of ethylenediamine diacetic acid or ethylenediamine triacetic acid with glycidyl(meth)acrylate, in particular the compounds described in WO 2013/066927. 2-Acetoacetoxyethyl methacrylate is the most preferred chelating monomer.

The monomer mixture may comprise 0 to 10 mol%, preferably 0 to 7 mol%, and more preferably 0 to 5 mol% of one or more chelating monomers.

The copolymers (a) preferably have a weight-average molar mass of 100 to 18000 kDalton (1 Dalton = 1 Da = 1 g/mol), more preferably 200 to 5000 kDa, and most preferably 250 to 3000 kDa.

The weight-average molar mass ($M_w$) of polymers is measured by asymmetric flow field-flow fractionation (AF4) in combination with a multi angle light scattering (MALS) and a refractive index (RI) detector (AF4/-MALS/RI). Separation is carried out on a 27.5 cm trapezoidal channel mounted on PEEK (polyether ether ketone) upper and lower blocks with a stainless-steel frit. A channel thickness spacer with a thickness of 490 $\mu$m is used. The accumulation wall is a regenerated cellulose membrane with a cut-off molar mass of 10000 Da. AF4 flow control can be performed with a Wyatt Eclipse 3 AF4 Separation System controller (Wyatt Technology, USA). A preferred MALS detector is a Dawn Heleos II detector (Wyatt Technology, USA) and a preferred RI detector is an Optilab Rex detector (Wyatt Technology, USA). The $dn \cdot dc^{-1}$ value used is chosen according to the respective polymer (0.084 $ml \cdot g^{-1}$ for PMMA and 0.064 $ml \cdot g^{-1}$ for PBA). An initial cross-flow of 3.0 $ml \cdot min^{-1}$ is used, which is exponentially decreased to 0.05 $ml \cdot min^{-1}$ over 42 minutes. After that, it is kept constant for another 13 minutes. The AF4/MALS/RI data are analyzed by using the ASTRA software version 6.1 (Wyatt Technology, USA). The absolute molar mass is calculated from the MALS/RI data using the Debye plot (with first-order Zimm formalism).

Optionally, transfer agents can be used to control the molecular weight of the copolymer (a). Preferred transfer agents are sulfur compounds, e.g., thiodiglycol, thioethanol, di-n-butyl sulfide, 2-mercaptoethanol, 1,3-mercaptopropanol, 3-mercaptopropane-1,2-diol, 1,4-mercaptobutanol and thioglycolic acid. Further preferred chain transfer agents are aldehydes, organic acids, e.g., formic acid, sodium or ammonium formate, and phosphorus compounds, e.g. sodium hypophosphite. If needed, transfer agents are used in an amount of 0.01 to 0.5 wt.%, based on the total amount of monomers.

The compositions of the present invention comprise the at least one copolymer (a) in dispersed form. Preferably, the at least one copolymer (a) is in the form of particles whose size can vary between a few nm and a few $\mu$m. Preferably, the copolymer has a number-average particle size of from 5 nm to 900 nm, and more preferably of from 30 to 300 nm. Polymer particles with a particle size below about 50 nm result in largely transparent copolymer dispersions. Dispersions comprising polymer particles with a diameter of a few $\mu$m are translucent and appear turbid or white.

The particle sizes are determined by dynamic light scattering, preferably using a Malvern Zetasizer (Nano Series, Malvern). For the analysis, samples of the dispersion are diluted with pure water (milli-Q water) to concentrations below $1 \cdot 10^{-3}$ wt.% so that unswollen particles are measured. The analysis is carried out at 25°C . Each sample is analyzed three times and the average value is calculated. The particle size is calculated according to the Stokes-Einstein equation.

The polymer dispersions, also called latex, are colloidal dispersions of polymer particles in a **water phase (b)**. They are stable over a long period of time. No sedimentation takes place. Synthetic polymer dispersions can be prepared by emulsion, miniemulsion, microemulsion, dispersion or suspension polymerization of corresponding monomers in the presence of a dispersant (emulsifier or suspending agent). Depending on the charge of the polymer particles and the type of dispersant, a distinction is made between cationically or anionically stabilized polymer dispersions. In cationic polymer dispersions, the dispersed polymer particles carry a positive charge. For instance, the polymers may comprise cationic groups, e.g., quaternary ammonium groups, and dispersions comprising polymers with basic nitrogen-containing monomers can be stabilized by addition of an acid. Alternatively, cationic dispersions can be prepared by using a cationic dispersant.

Polymerization is initiated by an initiator for the radical polymerization, preferably a thermal or redox initiator. Preferred thermal initiators are water soluble peroxides, hydroperoxides and azo compounds, such as 2,2'-azobis(isobutyramidine) dihydrochloride or 2,2'-azobis(N,N-dimethyleneisobutyramidine) dihydrochloride (both from Wako Chemical GmbH), which are radical initiators bearing a cationic group, 2,2'-azobis(4-methoxy-2,4-dimethylvaleronitrile) and 2,2-azobis(2,4-dimethylvaleronitrile). Further preferred thermal initiators are hydrogen peroxide and sodium or potassium persulfate.

Redox initiators systems comprise an oxidizing agent in combination with a reducing agent. Preferred oxidizing agents are the above thermal initiators and alkyl hydroperoxides such as t-butyl hydroperoxide. Preferred reducing agents are ascorbic acid and its derivatives/-salts, barbituric acid and its derivatives/salts, thiourea and its derivatives and sulfinic acids and its salts/derivatives.

Mostly preferred initiators are 2,2'-azobis(isobutyramidine) dihydrochloride and sodium or potassium persulfate.

The at least one initiator or initiator system is preferably used in an amount of 0.01 to 1.0 mol%, preferably 0.03 to 0.8 mol% and more preferably 0.04 to 0.5 mol%, based on the total amount of monomers.

The initiator may be added to the monomer mixture before it is emulsified in the aqueous phase. Preferably, the initiator is first dissolved or dispersed in water and then the solution or dispersion of the initiator is added to an emulsion of the monomer mixture in water.

Preferably, dispersions of the copolymer (a) are prepared by one of the following synthetic methods:

(I) Free-radical emulsion polymerization of a mixture of monomers (a1) and (a2) in the presence of a cationic surfactant.
(II) Free-radical emulsion polymerization of monomers (a1) and (a2) in the presence of a cationic or preferably nonionic surfactant and using a radical initiator bearing cationic groups.
(III) Free-radical emulsion polymerization of a mixture of monomers (a1), (a2) and at least one cationic monomer in the presence of a cationic or preferably nonionic surfactant.
(IV) Free-radical emulsion polymerization of a mixture of monomers (a1), (a2) and at least one basic nitrogen-containing monomer in the presence of a cationic or preferably nonionic surfactant, followed by the protonation of the nitrogen-containing monomer units with an acid, preferably with HCl.

In all instances, an antimicrobial monomer can be added, if desired. Acidic monomers must be processed according to methods (I) to (III) because they would react with the basic monomer used in method (IV). Methods (I) to (III) are preferred and methods (I) and (II) are more preferred. Zwitterionic monomers are processed according to method (III) using a zwitterionic monomer instead a cationic one.

In method (I), a cationic surfactant is preferably used, preferably in an amount of 0.01 to 1.0 wt.%, based on the total amount of monomers.

In method (II), a nonionic surfactant is preferably used, preferably in an amount of 0.01 to 2.0 wt.%. The radical initiator bearing cationic groups is preferably used in an amount of 0.01 to 0.5 wt.%, in each case based on the total amount of monomers.

In method (III), a nonionic surfactant is preferably used, preferably in an amount of 0.01 to 1.0 wt.%; and the at least one cationic monomer is preferably used in an amount of 0.1 to 15 mol.%, in each case based on the total amount of monomers.

In method (IV), a nonionic surfactant is preferably used, preferably in an amount of 0.01 to 1.0 wt.%, based on the total amount of monomers; and the at least one basic nitrogen-containing monomer is preferably used in an amount of 0.1 to 15 mol.%, based on the total amount of monomers. After the copolymerization step, the nitrogen-containing monomer units of the polymer are protonated by adding 10 to 100 mol% of an acid, preferably HCl, HBr or HNOs, based on the amount of basic nitrogen-containing monomer units.

Thus, the (meth)acrylic copolymer dispersions (latexes) are preferably synthesized by free-radical emulsion polymerization of a mixture of emulsified monomers in water, using a water or oil soluble initiator and an emulsifier/surfactant. The emulsion polymerization processes can be carried out as batch polymerization, in which all ingredients are added to the reaction vessel at the beginning of the reaction, or as semi-continuous or semi-batch polymerization, in which the monomers are added continuously or stepwise, in neat form or in the form of an emulsion. The polymerization can also be performed as a continuous process, where all ingredients are continuously added to one part of the reactor system, and partially or completely converted latex is continuously removed from another part.

In all instances, the emulsion polymerization processes preferably comprise a particle nucleation stage and a particle growth stage. These stages can be performed consecutively or simultaneously. A seed latex can be used to obviate the particle nucleation stage. The seed latex can be prepared in a separate reaction or by polymerization *in situ*.

Preferably, the polymerization is carried out in a batch or semibatch process in which a mixture of monomers, surfactant, a sufficient amount of water is stirred in a reactor equipped with a condenser, a nitrogen inlet and a mechanical stirrer, and then a radical initiator is added. The polymerization mixture is preferably stirred under a nitrogen atmosphere, e.g., at 50 to 1000 rpm (revolutions per minute), and is additionally heated if a thermal initiator is used. In process (IV), a suitable amount of acid is added after the polymerization step is completed.

The composition of the copolymer is determined by the type and ratio of monomers used as well as by the copolymerization parameters. The amount of water determines the final solid content of the latex. The water content of the final latex is preferably adjusted to be in the range of 5 to 50 wt.%, more preferably 10 to 45 wt.%, and most preferably 15 to 40 wt.%, based on the total dispersion. The polymerization time depends on the polymerization conditions, and is preferably within a range of 0.2 to 20 h, more preferably 0.5 to 15 h, and most preferably 1 to 10 h. It is chosen to achieve the highest as possible monomer conversion. To optimize monomer conversion, it may be advantageous to perform a post-polymerization step, for example, by adding an additional amount of initiator and/or increasing the polymerization temperature.

The overall monomer conversion $X_{overall}$ is determined gravimetrically by measuring the non-volatile fraction of the dispersion. The overall monomer conversion is calculated from the amount of polymer at time t ($m_{pol}$(t); t = end of the reaction) divided by the total amount of monomer fed ($m_{mon,total}$):

$$X_{overall} = m_{pol}(t)/m_{mon,total}$$

Residual monomers can be measured by gas chromatography (GC), e.g., with a GC-14A chromatograph (Shimadzu) equipped with a flame ionization detector and a polar PEG-column modified with nitroterephthalic acid (BP21 FFAP (Free Fatty Acid Phase) column from SGE Analytical Science; length 25 m, inner diameter of 0.53 mm, film thickness 0.5 $\mu$m).

The cationic polymer dispersions of the present invention exhibit high colloidal stability, i.e., they do not sediment or flocculate. A measure of colloidal stability is the Zeta-potential of the copolymer particles. The Zeta potential can be measured using the Zetasizer Nano (Malvern). For the analysis, samples of the copolymer dispersion are diluted with pure water (milli-Q water) to concentrations below $1 \cdot 10^{-3}$ wt.% so that unswollen particles are measured. The analysis is carried out at 25°C and each sample is analyzed three times and the average value is calculated.

The dispersed copolymer particles of the present invention preferably have a glass transition temperature of -20 to 55°C, more preferably of -10 to 50°C and most preferably of -5 to 40°C. The glass-transition temperature of the copolymer particles is important for the film-forming properties of the copolymer dispersion. The capability of the polymer dispersions to form a thin, continuous film upon evaporation of water (dry up) is important for their application as a varnish or coating. The so-called minimum film formation temperature (MFFT) is the lowest temperature at which a transparent film is formed by a polymer dispersion. The MFFT depends on the glass-transition temperature of the copolymer particles of the dispersion. The MFFT is preferably determined using an MFFT Bar instrument (MFFT - 90, Rhopoint instruments Ltd., St. Leonards-on-Sea, UK). A wet layer of the dispersion with 90 $\mu$m thickness is placed on a temperature gradient bar with a temperature ranging from 5 to 85°C. The MFFT of films prepared with the copolymer dispersion of the present invention is preferably in the range of 0 to 60°C, more preferably 2.5 to 50°C, and most preferably 5 to 40°C.

The copolymer dispersions according to the present invention comprise at least one **surfactant (c)**. The surfactant (c) colloidally stabilizes the monomer emulsion and the final copolymer dispersion. Colloidal stabilization of the copolymer particles can be achieved by ionic and non-ionic surfactants. The surfactant molecules can be physically or chemically bound to the surface of copolymer particles. For instance, the polymerization of nonionic monomers (a1) and (a2) results in the formation of nonionic copolymer particles. The chemically or physically binding of a cationic surfactant gives these particles a positive charge, which causes cationic stabilization. For cationic copolymer particles, non-ionic surfactants can be used, which have primarily the function of an emulsifier.

The at least one surfactant is selected from the group consisting of non-ionic surfactants, cationic surfactants, amphoteric surfactants, and polymeric surfactants. Nonionic surfactants and their mixtures with cationic, amphoteric, and polymeric surfactants are preferred. For nonionic copolymers, the use of a cationic surfactant is mandatory.

Preferred non-ionic surfactants are alkyl ethoxylates, ethylene oxide-propylene oxide block copolymers, and polysorbates. Preferred alkyl ethoxylates are (i) ethoxylated fatty alcohols of the formula $CH_3(CH_2)_nO(CH_2CH_2O)_xH$, with n = 8 to 71, more preferably 10 to 35, and x = 5 to 200, more preferably 10 to 40; and (ii) ethoxylated fatty acids of the formula $CH_3(CH_2)_nCOO(CH_2CH_2O)_xH$, with n = 8 to 71, more preferably 10 to 35, and x = 5 to 200, more preferably 10 to 40. Preferred ethylene oxide-propylene oxide block copolymers are copolymers with a molecular weight from 1000 to 15000 g/mol. Polysorbates are esters of polyethoxylated sorbitan with fatty acids, preferably with dodecanoic acid, oleic acid or hexadecanoic acid.

Preferred cationic surfactants are alkyltrimethyl, dialkyldimethyl or alkylaryldimethyl ammonium compounds with different counterions, such as dimethyldioctadecyl-ammonium bromide, hexadecyltrimethylammonium bromide, octenidine dihydrochloride and other salts of octenidine, cetyltrimethylammonium bromide, cetylamine hydrofluoride (Hetaflur), cetylamine hydrochloride, cetylpyridinium chloride (CPC), dodecyltrimethylammonium chloride, dimethyldidodecylammonium bromide or benzyldimethylhexadecylammonium chloride. Further preferred cationic surfactants are fatty amine salts, fatty diamine salts and imidazolium salts.

Preferred amphoteric (zwitterionic) surfactants are alkylbetaines and alkylamidobetaines. Preferred alkylbetaines are cetyl, lauryl, oleyl and stearyl betaine. Preferred alkylamidobetaines are cocamidopropyl betaine, oleamidopropyl betaine and lauryl-amidopropyl betaine.

Polymeric surfactants or surface active oligomers can also be used. These are random or block copolymers of hydrophobic monomers and monomers containing carboxylic acid moieties. Preferred hydrophobic monomers are methyl styrene, styrene, and methyl methacrylate), and preferred carboxylic acid groups containing monomers are acrylic and methacrylic acid. The polymeric surfactants preferably have a molecular weight in the range of 4000 to 40000 g/mol and more preferably 5000 to 20000 g/mol.

Preferred polymeric non-ionic surfactants are polyethylene oxide-polypropylene oxide block copolymers.

The surfactants can contain one or more, preferably one, olefinically unsaturated group, that can participate in a free radical polymerization. Such surfactants can be chemically linked to the surface of the copolymer particles. Polymerizable surfactants are also called surfmers. Surfmers can bear ionic or non-ionic moieties, with non-ionic surfmers being preferred according to the invention.

Preferred non-ionic surfmers are vinyl and allyl polyoxyethylene glycol ethers or allyl polyoxypropylene glycol ethers with 10 to 30, preferably 20 oxyalkylene (EO or PO) units, allyl phenylpolyol ether sulfates and allyl phenylpolyol ether ammonium salts, each with 10 to 30 EO or PO units.

The at least one surfactant (c) is preferably added to the aqueous phase (b) together with the monomer mixture (a) in order to achieve a uniform dispersion of the monomer mixture in the aqueous phase. An additional amount of one or more surfactants may be added after emulsion polymerization is complete. Preferably, one or more nonionic surfactants are added if this is required to stabilize the dispersion during or after the addition of further components. The total amount of surfactants is within the above defined range of 0.5 to 6.0 wt.%, preferably 0.6 to 5.5 wt.%, and more preferably 0.7 to 5.0 wt.% based on the total weight of the dispersion.

The compositions of the present invention comprise at least one **organic or inorganic fluoride source (d)**.

Preferred fluorides are sodium fluoride, potassium fluoride, ammonium fluoride, ammonium bifluoride, sodium monofluorophosphate, potassium monofluorophosphate, ammonium hexafluorosilicate, magnesium hexafluorosilicate, potassium hexafluorosilicate, ammonium hexafluorotitanate, ammonium hexafluoroaluminate, zirconium fluoride, tetra-n-butylammonium dihydrogentrifluoride (TBAF-3), rubidium fluoride, cesium fluoride, potassium bifluoride ($KHF_2$), silver(I) fluoride, tin(II) fluoride, and the amine fluorides Olaflur (N,N,N'-tris(2-hydroxyethyl)-N'-octadecyl-1,3-diaminopropane dihydrofluoride) and Dectaflur (9-octadecenylamine hydrofluoride). Particularly preferred fluorides are ammonium fluoride, ammonium bifluoride, sodium fluoride, potassium fluoride and tetra-n-butylammonium dihydrogentrifluoride (TBAF-3).

The fluorides are preferably added to the dispersion after polymerization is complete.

The fluoride source is an essential component of dental fluoride varnishes. However, fluoride ions have been found to reduce the stability of aqueous polymer dispersions. This effect is concentration-dependent, making it difficult to produce stable dispersions without organic solvents that contain high fluoride concentrations, as required especially for professional use. According to the present invention, it was surprisingly found that stable dental varnishes with high fluoride content can be prepared by using cationic polymer dispersions comprising a dispersed copolymer as defined above.

According to one embodiment, the compositions of the present invention comprise at least one **antimicrobial compound.** The antimicrobial compound may be an antimicrobially active surfactant. Preferred surfactants (c) which have an antimicrobial active are cetylpyridinium tetrachlorozincate, octenidine dihydrochloride, cetylamine hydrofluoride (Hetaflur), cetylamine hydrochloride, more preferably dodecyltrimethyl-lammonium chloride, most preferably cetylpyridinium chloride, and mixtures thereof. Antimicrobially active surfactants are preferably used in an amount of at least 0.001 wt.%, more preferably of at least 0.01 wt.% and most preferably of at least 0.1 wt.%, provided that the total amount of surfactants in within the above-mentioned ranges.

Alternatively or in addition to an antimicrobially active surfactant, the polymer dispersions according to the invention can comprise one ore more low-molecular weight antimicrobial agents (e). According to the present invention, a low-molecular weight antimicrobial agent is a compound having a molecular weight of < 1500 g/mol, preferably of < 1250 g/mol, and more preferably of < 1000 g/mol.

Preferred low-molecular weight antimicrobial compounds (e) are quarternary ammonium compounds, such as octenidine, benzalkonium chloride and benzethonium chloride; biguanides, such as chlorhexidine (and salts thereof), chlorhexidine digluconate, chlorhexidine diacetate, chlorhexidine dihydrochloride, alexidine and salts thereof; azol-antifungals, such as clotrimazole, fluconazole, iltraconazole, ketoconazole, miconazole, voroconazole, posaconazole and oteseconazole; parabenes (esters of p-hydroxybenzoic acid), such as methyl, ethyl, propyl, and butyl parabenes and salts thereof, and other antimicrobials such as sorbic acid and salts thereof, glyoxal, glutaraldehyde, diazolinidyl urea, imidazolinidyl urea, ethacridin lactate, pirocton-olamin, hexetidin, triclosan and aetheric oils, such as thymol or eugenol. Further antimicrobials are drug antibiotics, e.g. aminoglycosides such as, amikacin, gentamicin, kanamycin, neomycin, netilmicin, tobramycin, paromomycin, streptomycin and spectinomycin (Bs); ansamycins, such as geldanamycin, herbimycin and rifaximin; carbapenems, such as ertapenem, doripenem, imipenem/cilastatin and meropenem; glycol peptides, such as teicoplanin, vancomycin, telavancin, dalbavancin and oritavancin; lincosamides, such as clindamycin and lincomycin; macrolides, such as azithromycin, clarithromycin, erythromycin, roxithromycin, telithromycin, spiramycin and fidaxomicin; nitrofurans, such as furazolidone and nitrofurantoin; oxazolidinones, such as linezolid, posizolid, radezolid or torezolid; penicillins, such as amoxicillin, ampicillin, azlocillin, dicloxacillin, flucloxacillin, mezlocillin, methicillin, nafcillin, oxacillin, penicillin, piperacillin, temocillin and ticarcillin; quinolones/fluoroquinolones, such as ciprofloxacin, enoxacin, gatifloxacin, gemifloxacin, levofloxacin, lomefloxacin, moxifloxacin, nadifloxacin, nalidixic acid, norfloxacin, ofloxacin, trovafloxacin, grepafloxacin, sparfloxacin, and temafloxacin; sulfonamides, such as mafenide, sulfacetamide, sulfadiazine, silver sulfadiazine, sulfadimethoxine, sulfamethizole, sulfamethoxazole, sulfasalazine and sulfisoxazole; tetracyclines, such as demeclocycline, doxycycline, metacycline, minocycline, oxytetracycline and tetracycline; drugs against mycobacteria, such as clofazimine, dapsone, capreomycin, cycloserine, ethambutol, ethionamide, isoniazid, pyrazinamide, rifampicin, rifabutin, rifapentine and streptomycin; and other compounds such as arsphenamine, chloramphenicol, fosfomycin, fusidic acid, metronidazole, mupirocin, platensimycin, quinupristin/dalfopristin, thiamphenicol, tigecycline (Bs), and tinidazole.

Particularly preferred low-molecular weight antimicrobial agents (e) are octenidine, chlorhexidine and salts thereof, chlorhexidine digluconate, chlorhexidine diacetate, phthalimidoperoxycaproic acid (PAP) and mixtures thereof. Most preferred antimicrobials are chlorhexidine and salts thereof, chlorhexidine digluconate, chlorhexidine diacetate, and mixtures thereof. One or more low-molecular weight antimicrobial compounds can be used.

The compositions of the present invention preferably may comprise from 0 to 3.0 wt.%, more preferably from 0.1 to 2.0 wt.% and most preferably from 0.2 to 1.5 wt.% of one or more low-molecular weight antimicrobial compounds (e).

The compositions of the present invention can preferably comprise one or more **further components** selected from humectants, oxidizing agents and other additives such as sweeteners, colorants, flavors, pH-regulators, soothing agents, and thickeners. Further components are added with stirring to the copolymer dispersion preferably after completion of polymerization.

Preferred **humectants** are 1,2-propylen glycol, ethylene glycol, diethylene glycol, more preferably polyethylene glycol, and most preferably xylitol, sorbitol, glycerol, and mixtures thereof.

Although many humectants are liquid organic compounds, they are not considered organic solvents within the meaning of the present invention and are not excluded from the claimed compositions. Unlike organic solvents, they are low-volatility viscous liquids which do not readily evaporate at room temperature, and which are therefore usually not used for the preparation of dental varnishes.

The compositions of the present invention may preferably comprise from 0 to 30 wt.%, more preferably from 0 to 25 wt.% and most preferably from 0 to 20 wt.% of one or more humectants.

Preferred **oxidizing agents** are hydrogen peroxide, carbamide peroxide, chloramine T (sodium-N-chloro-4-methylbenzolsulfonamide), sodium chlorate and phthalimidoperoxycaproic acid (PAP). While certain oxidizing agents such as hydrogen peroxide have an antimicrobial effect, they are mainly added to a achieve a bleaching effect. Preferred oxidizing agents are hydrogen peroxide and carbamide peroxide.

The compositions of the present invention preferably can comprise from 0 to 30 wt.% of one or more oxidizing agents, more preferably from 0 to 25 wt.%, most preferably from 0 to 20 wt.%.

According to a particularly preferred embodiment, the compositions of the present invention can comprise from 0 to 16 wt.% of one or more oxidizing agents. They preferably contain from 1 to 16 wt.%, more preferably from 3 to 13 wt.% and even more preferably from 4 to 12 wt.% and most preferably from 4 to 6 wt.% of one or more oxidizing agents.

Preferred **sweeteners** are acesulfame K, advantame, aspartame, cyclamates, isomalt, saccharins, sucralose, thaumatin, neohesperidine DC, steviol glycosides, neotame, salts of aspartame and acesulfame.

Preferred **colorants** are those listed in the "REGULATION (EC) No 1333/2008 OF THE EUROPEAN PARLIAMENT AND OF THE COUNCIL of 16 December 2008" on food additives, numbers E100-E180, and substances listed in the INCI (International Nomenclature of Cosmetic Ingredients) with the function "colorants".

Preferred **flavors** are natural and artificial flavorings or flavoring extracts with a smell/taste of peppermint, orange, strawberry, vanilla, melon etc. Suitable flavorings are listed in "COMMISSION DECISION of 23 Feb. 1999 adopting a register of flavoring substances used in or on foodstuffs" drawn up in application of Regulation (EC) No. 2232/96 of the European Parliament and of the Council of 28 October 1996 (1999/ 217/EC).

Preferred **pH-regulators** are those listed in the INCI (International Nomenclature of Cosmetic Ingredients) with the function "buffering".

Preferred **soothing agents** are D-panthenol, bisabolol or allantoin; and preferred thickeners are water soluble, anionic cellulose ethers, e.g. hydroxypropyl cellulose, hydroxyethyl cellulose.

The compositions of the present invention preferably comprise a total amount of from 0.01 to 10 wt.%, more preferably from 0.1 to 8.0 wt.% and most preferably from 0.2 to 6.0 wt.% of one or more additives (sweeteners, colorants, flavors, pH-regulators, soothing agents, or thickeners).

The copolymer dispersions of the present invention are particularly suitable as dental materials and especially for the production of therapeutic and non-therapeutic dental varnishes or coatings, in particular for the fluoridation of teeth.

For use, the areas of the tooth or teeth to be treated are preferably cleaned before the varnish is applied. This removes tartar (calculus) and other deposits on the teeth. Professional tooth cleaning is particularly preferred.

The tooth or teeth to be treated are then preferably dried. This can be done with cotton rolls, cotton wool wads and, if necessary, a saliva ejector or an air syringe. The creation of a dry working area can be simplified by using a cheek retractor. This facilitates free access to the tooth surfaces.

Next, the dental varnish is applied to the surface of at least one tooth. Preferably, the varnish is applied to the entire natural set of teeth to achieve a comprehensive treatment of the teeth. However, the varnish can also easily be applied only to certain areas that are to be treated, especially if no professional dental cleaning has been carried out beforehand.

To apply the dental varnish, a small portion is preferably placed in a suitable container, for example, a dappen dish or similar container. Then, the dental varnish is applied in a thin layer to the tooth surfaces with a brush and left to dry, preferably for one to several minutes. Drying can be expedited by using an air syringe. When the varnish has dried, the cotton rolls and the cheek retractor, if used, can be removed. Preferably, patients are advised not to eat or drink for one hour after the treatment.

The copolymer dispersions according to the invention are particularly suitable for the remineralization of teeth and for the treatment or prophylaxis of caries. Due to their high fluoride content, they are especially suitable for therapeutic use by the dentist, in particular for use in a method for the treatment and/or prevention of caries and initial caries lesions (initial caries), of white spots, of dental erosion, for hardening damaged teeth and for the restoration of hard tooth tissue. However, the dental varnishes according to the invention can also be used for the non-therapeutic treatment of teeth.

Compositions for use as dental varnishes or coatings preferably comprise:

(a) 5 to 41 wt.%, preferably 7 to 35 wt.%, and more preferably 15 to 30 wt.% of at least one copolymer (a) as defined above,

(b) 50 to 94 wt.%, preferably 60 to 91 wt.%, and more preferably 65 to 83 wt.% of water,

(c) 0.5 to 6.0 wt.%, preferably 0.6 to 5.5 wt.%, and more preferably 0.7 to 5.0 wt.%. of at least one surfactant,

(d) 0.3 to 3.0 wt.%, preferably 0.5 to 2.5 wt.% and more preferably 1.0 to 2.0 wt.% of at least one inorganic or organic fluoride source (calculated as $F^-$),

(e) 0 to 3.0 wt.%, preferably 0.1 to 2.0 wt.% and more preferably 0.2 to 1.5 wt.% of one or more low-molecular weight antimicrobial compounds,

(f) 0 to 30 wt.%, preferably 0 to 25 wt.% and more preferably 0 to 20 wt.% of one or more humectants,

(g) 0 to 16 wt.%, preferably 1 to 16 wt.% and more preferably 3 to 13 wt.% of one ore more oxidizing agents, and

(h) optionally 0.01 to 10 wt.%, preferably 0.1 to 8.0 wt.% and more preferably 0.2 to 6.0 wt.% of at least one additive selected from sweeteners, colorants, flavors, pH-regulators, soothing agents, thickeners or a mixture thereof,

in each case in relation to the total weight of the vanish.

In a particularly preferred embodiment of the invention, the amounts of components (a) to (h) add up to 100 %.

The invention is explained in more detail below with reference to examples.

## EXAMPLES

### Example 1

*General procedure for the preparation of a copolymer dispersion by two-step seeded emulsion polymerization*

Step 1 (Preparation of seed polymer): In a flask, 300 ml water was purged with nitrogen, 0.50 g of the ionic surfactant cetylpyridinium chloride (CPC, which corresponds 0.125 wt.% in the final dispersion) and 8.40 g of a nonionic surfactant (Disponil® AFX3070; modified fatty alcohol ethoxylate; BASF SE, Ludwigshafen, Germany; which corresponds 2.0 wt.% in the final dispersion) were added and stirred for 10 min. Subsequently, 4.63 g n-butyl acrylate (BuA) and 3.82 g methyl methacrylate (MMA) were added to the solution under stirring (500 rpm) and the white emulsion was heated to 70°C and stirred for 15 min. The initiator solution (0.60 g 2,2'-azobisisobutylamidinium chloride (AIBA) dissolved in 12 mL water) was dropped to the emulsion and the mixture was stirred at 70°C for 30 min.

Step 2 (Growth stage): A monomer mixture of 44.60 g BuA and 36.74 g MMA was added under stirring (500 rpm) to the seed polymer via a dropping funnel at 70°C over a period of 3 h. After finished addition, the mixture was heated to 80°C and stirred for another hour (500 rpm) to obtain the final (meth)acrylate copolymer latex as white, slightly transparent dispersion **D1** with the following properties: Particle size: 50 nm, zeta potential: 23 mV, and pH of dispersion: 3.5. The molar ratio of MMA to n-BuA was 51:49 in the copolymer. For a copolymer with this monomer ratio a glass transition temperature of -3°C was determined.

**Example 2**

*Preparation of a dispersion of n-butyl acrylate/methyl methacrylate copolymer particles with a cationic and a nonionic surfactant (dispersion D2)*

According to the general procedure of Example 1, a seed polymer was prepared from 0.50 g CPC (0.125 wt.% in the final dispersion), 8.40 g Tween 20 (polyethylene glycol sorbitan monolaurate: Merck, 2.0 wt.% in the final dispersion), 4.63 g BuA, 3.82 g MMA, 0.60 g AIBA and 312 mL water.

According to the general procedure, particle growth was induced by adding a mixture of 44.60 g BuA and 36.74 g MMA. The final (meth)acrylate copolymer latex was obtained as white, slightly transparent dispersion **D2** with the following properties: Particle size: 50 nm, zeta-potential: 40 mV. The molar ratio of MMA to n-BuA was 51:49 in the copolymer.

**Example 3**

*Preparation of a dispersion of n-butyl acrylate/methyl methacrylate copolymer particles with a cationic and a nonionic surfactant (dispersion D3)*

According to the general procedure of Example 1, a seed polymer was prepared from 0.50 g CPC (0.125 wt.% in the final dispersion), 16.80 g Tween 20 (4.0 wt. in the final dispersion), 6.22 g BuA, 2.61 g MMA, 0.60 g AIBA and 312 mL water.

According to the general procedure, particle growth was induced by adding a mixture of 59.65 g BuA and 25.01 g MMA. The final (meth)acrylate copolymer latex was obtained as white, slightly transparent dispersion **D3** with the following properties: Particle size: 46 nm, zeta-potential: 30 mV. The molar ratio of MMA to n-BuA was 35:65 in the copolymer. For a copolymer with this monomer ratio a glass transition temperature of -20°C was determined.

**Example 4**

*Preparation of a dispersion of n-butyl acrylate/methyl methacrylate/methacrylic acid copolymer particles (dispersion 04)*

According to the general procedure of Example 1, a seed polymer was prepared from 0.50 g CPC (0.125 wt.% in the final dispersion), 8.40 g Disponil® AFX3070 (2.0 wt. in the final dispersion), 6.54 g BuA, 2.62 g MMA, 0.14 g methacrylic acid, 0.60 g AIBA and 312 mL water.

According to the general procedure, particle growth was induced by adding a mixture of 59.15 g BuA and 23.49 g MMA and 1.25 g methacrylic acid (MAA). The final (meth)acrylate copolymer latex was obtained as white, slightly transparent dispersion **D4** with a pH-value of 3.7.

**Example 5**

*Preparation of a dispersion of n-butyl acrylate/methyl methacrylate/2-[(propoxycarbonyl)amino]ethyl methacrylate copolymer particles (dispersion D5)*

According to the general procedure of Example 1, a seed polymer was prepared from 0.50 g CPC (0.125 wt.% in the final dispersion), 8.40 g Disponil® AFX3070 (2.0 wt. in the final dispersion), 5.00 g BuA, 2.31 g MMA, 3.40 g 2-[(propoxycarbonyl)amino]ethyl methacrylate, 0.60 g AIBA and 312 mL water.

According to the general procedure, particle growth was induced by adding a mixture of 45.60 g BuA and 21.30 g MMA and 30.60 g 2-[(propoxycarbonyl)amino]ethyl methacrylate. The final (meth)acrylate copolymer latex was obtained as white, slightly transparent dispersion **D5**.

**Example 6**

*Preparation of a dispersion of n-butyl acrylate/styrene copolymer particles (dispersion D6)*

According to the general procedure of Example 1, a seed polymer was prepared from 0.50 g CPC (0.125 wt.% in the final dispersion), 8.40 g Disponil® AFX3070 (2.0 wt. in the final dispersion), 4.80 g BuA, 3.90 g styrene, 0.60 g AIBA and 312 mL water.

According to the general procedure, particle growth was induced by adding a mixture of 45.77 g BuA and 37.20 g styrene. The final acrylate/styrene copolymer latex was obtained as white, slightly transparent dispersion **D6** with the following properties: Particle size: 40 nm. The molar ratio of styrene to n-BuA was 50:50 in the copolymer.

**Example 7**

*Preparation of a dispersion of n-butyl acrylate/methyl methacrylate/([2-(methacryloyloxy)ethyl] trimethylammonium chloride copolymer particles*

According to the general procedure of Example 1, a seed polymer was prepared from 8.40 g Disponil® AFX3070, 4.54 g BuA, 3.96 g MMA, 1.91 g MATMAC ([2-(methacryloyloxy)ethyl] trimethylammonium chloride, 75% solution in water), 0.60 g AIBA and 225 mL water.

According to the general procedure, particle growth was induced by adding a mixture of 40.96 g BuA and 35.59 g MMA. This monomer mixture was added simultaneously with a solution of 9.03 g MATMAC (75% solution in water) in 75 mL water over a period of 75 min. The final (meth)acrylate copolymer was obtained as white, slightly transparent dispersion.

**Example 8**

*Preparation of a dispersion of n-butyl acrylate/methyl methacrylate copolymer particles*

Preparation of a batch copolymer: In a reactor equipped with a nitrogen inlet, a condenser and a syringe pump, 298 ml water was purged with nitrogen, 0.28 g of CPC (which corresponds 0.06 wt.% in the final dispersion) and 7.80 g of Disponil® AFX3070 (which corresponds 2.0 wt.% in the final dispersion) were added and stirred for 10 min. Subsequently, 49.2 g BuA and 40.2 g MMA were added to the solution under stirring (300 rpm) and the emulsion was heated to 70°C and stirred for 15 min. The initiator solution (0.14 g (70% in water) tert-butyl hydroperoxide (TBHP)) was added and the feeding of 0.2 g of ascorbic acid (reducing agent of the redox initiator system) dissolved in 2 ml of water was started. The acid was fed for 3 h and the mixture was stirred 1 h more at 80°C to obtain the final (meth)acrylate copolymer latex as white dispersion with the following properties: Particle size: 100 nm, zeta potential: 43 mV, and pH of dispersion: 3.4. The molar ratio of MMA to n-BuA was 51:49 in the copolymer. For a copolymer with this monomer ratio a glass transition temperature of 7°C was determined. The molecular weight determined with asymmetric flow field flow fractionation method was 18000 kDa.

**Example 9**

*Preparation and properties of fluoride-containing varnishes*

0.30 g sodium fluoride (3 wt.%) were added to 9.70 g each of dispersions **D1**, **D3** and **D5** with stirring until the sodium fluoride is completely dissolved. All formed fluoride varnishes were stable for months.

Adhesion tests of films formed from the prepared fluoride varnishes on enamel were performed on bovine teeth. The bovine teeth were embedded in a polymer block and then the enamel was exposed by grinding. Before applying the fluoride varnish, the tooth surface was polished with abrasive paper (4000 Grid). A thin layer of varnish was applied with a brush (VivaBrush G, Ivoclar Vivadent AG) and then dried with a gentle stream of air. The prepared test specimens were stored for 4 h in water (contains a small amount of rose bengal as a dye). After drying the test specimens in a light air stream, the film quality was assessed and the adhesion to enamel is determined semi-quantitatively. For this purpose, an adhesive tape (tesafilm®, tesa SE, Norderstedt, Germany) was applied to the test specimen with the exposed tooth surface. The tape was then slowly pulled off the tooth at an acute angle. A visual assessment of the adhesion was made. All prepared fluoride varnishes showed an excellent adhesion on enamel. That means very little or nothing of the films were detached or to be scratched away with a metal spatula.

**Example 10**

*Preparation and properties of fluoride-containing varnishes with the antimicrobial agent CHX*

0.20 g sodium fluoride (2 wt.%) and 0.35 g of a 20 wt.% chlorhexidine digluconate (CHX) solution in water (3.5 wt.% CHX) were added to 9.45 g each of dispersions **D1** to **D6** with stirring until the sodium fluoride was completely dissolved. All formed fluoride varnishes were stable at least for 2 years (= observation period). Adhesion tests of dispersion films were performed according to Example 9. All prepared fluoride/CHX varnishes showed an excellent adhesion on enamel.

**Claims**

1. A cationic polymer dispersion comprising:

    (a) 5 to 41 wt.%, preferably 7 to 35 wt.%, and more preferably 15 to 30 wt.%. of at least one copolymer,
    (b) 50 to 94 wt.%, preferably 60 to 91 wt.%, and more preferably 65 to 83 wt.% of water, and
    (c) 0.5 to 6.0 wt.%, preferably 0.6 to 5.5 wt.%, and more preferably 0.7 to 5.0 wt.% of at least one surfactant,
    **characterized in that** it comprises
    (d) 0.3 to 3.0 wt.%, preferably 0.5 to 2.5 wt.% and more preferably 1.0 to 2.0 wt.% of at least one organic and/or inorganic fluoride source (calculated as $F^-$) and
    contains essentially no low-boiling organic solvent,

    all percentages by weight being based on the total weight of the dispersion.

**2.** The dispersion of claim 1, wherein the at least one copolymer (a) is a copolymer which is obtainable by free-radical copolymerization of a monomer mixture comprising:

(a1) 5 to 70 mol%, preferably 10 to 65 mol% and more preferably 15 to 60 mol% of at one least rigid (meth)acrylate or vinyl monomer and
(a2) 30 to 95 mol%, preferably 35 to 90 mol%, and more preferably 40 to 85 mol% of at least one flexible (meth)acrylate or vinyl monomer, wherein rigid monomers are (meth)acrylate monomers that form homopolymers with a glass transition temperature ($T_G$) of from 30°C to 130°C, and flexible monomers are (meth)acrylate or vinyl monomers that form homopolymers with a glass transition temperature ($T_G$) of from -70°C to 20°C, and wherein all mole percentages being based on the total molar amount of monomers.

**3.** The dispersion of claim 1 or 2, wherein the **monomer (a1)** is selected from methyl methacrylate, phenyl, naphthyl, benzyl, adamantyl (meth)acrylate, isobornyl (meth)acrylate, 2-[(methoxycarbonyl)amino] ethyl (meth)acrylate, 2-[(propoxycarbonyl)amino] ethyl (meth)acrylate, 2-[(isopropoxycarbonyl)-amino]ethyl (meth)acrylate, 2-[(butoxycarbonyl)amino] ethyl (meth)acrylate, 2-[(hexyloxycarbonyl)amino] ethyl (meth)acrylate, 2-[(cyclohexyloxycarbonyl)-amino]ethyl (meth)acrylate, 2-[(benzyloxycarbonyl) amino]ethyl (meth)acrylate, 2-[(ethylcarbamoyl) oxy]ethyl (meth)acrylate, 2-[(propylcarbamoyl)oxy] ethyl (meth)acrylate, 2-[(isopropylcarbamoyl)oxy] ethyl (meth)acrylate, 2-[(hexylcarbamoyl)oxy]ethyl (meth)acrylate, 2-[(benzylcarbamoyl)oxy]ethyl (meth)-acrylate, 2-[(2-tetrahydrofurfuryloxycarbonyl)amino]ethyl (meth)acrylate, 2-[(2-oxo-1,3-dioxolan-4-yl)methoxycarbonylamino]ethyl (meth)acrylate, (meth)-acrylic acid 2-(furan-2-yl-methoxycarbonylamino)ethyl ester, (1,3-dioxolan-2-on-4-yl) methyl (meth)acrylate, 2-phenoxyethyl (meth)acrylate, 2-(o-biphenyl-oxy)ethyl (meth)acrylate, 2-hydroxy-3-phenoxypropyl (meth)acrylate, 2-phenoxypropyl (meth)acrylate, 2-(p-cumylphenoxy)ethyl (meth)acrylate, tricylodecane (meth)acrylate, tricyclodecanemethyl (meth)acrylate and 1,7,7-trimethylbicyclo[2.2.1]heptan-2-yl (meth)acrylate, styrene, p-methylstyrene, p-ethylstyrene, p-methoxystyrene, $\alpha$-methyl styrene, divinyl benzene, acrylonitrile or a mixture thereof and/or
the **monomer (a2)** is selected from alkyl or cycloalkyl (meth)acrylates, n-butyl methacrylate, n-pentyl methacrylate, n-hexyl methacrylate, 2-ethylhexyl methacrylate, n-octyl methacrylate, n-dodecyl methacrylate, tetradecyl methacrylate, hexadecyl methacrylate, the corresponding acrylates, hydroxyalkyl (meth)acrylates, 2-hydroxyethyl acrylate, 2-octyl acrylate, 2-hydroxypropyl acrylate, vinyl acetate, the vinyl esters of saturated monocarboxylic acids with highly branched structure and 5 to 12, preferably 9 or 10, carbon atoms, the vinyl esters of neodecanoic acid and neononanoic acid, or a mixture thereof.

**4.** The dispersion of claim 3, wherein the monomer (a1) is selected from methyl methacrylate, benzyl methacrylate, styrene, or a mixture thereof, and the monomer (a2) is selected from n-butyl acrylate, n-hexyl acrylate, 2-ethylhexyl acrylate or a mixture thereof.

**5.** The dispersion according to any one of claims 2 to 4, wherein the monomer mixture in addition of monomer (a1) and (a2) comprises at least one additional radically polymerizable monomer that is selected from basic nitrogen-containing monomers, cationic monomers, zwitterionic monomers, antimicrobial monomers and/or adhesive monomers.

**6.** The dispersion according to any one of claims 1 to 5, wherein the at least one surfactant is selected from the group consisting of non-ionic surfactants, cationic surfactants, amphoteric surfactants, and polymeric surfactants, preferably non-ionic surfactants, and their mixtures with cationic, amphoteric, and polymeric surfactants.

**7.** The dispersion according to any one of claim 1 to 6, wherein the copolymer (a) has a weight-average molar mass (Mw) of 100 to 18000 kDa, preferably 200 to 5000 kDa, and more preferably 250 to 3000 kDa.

**8.** The dispersion according to any one of claims 1 to 7, wherein the copolymer (a) has a number-average particle size in a range of from 5 nm to 900 nm, and preferably from 30 to 300 nm.

**9.** The dispersion according to any one of claims 1 to 8, wherein the copolymer (a) is a copolymer obtainable by

(I) free-radical emulsion polymerization of a mixture of monomers (a1) and (a2) in the presence of a cationic surfactant,
(II) free-radical emulsion polymerization of monomers (a1) and (a2) in the presence of a cationic or preferably nonionic surfactant and using a radical initiator bearing cationic groups,
(III) free-radical emulsion polymerization of a mixture of monomers (a1), (a2) and at least one cationic monomer in the presence of a cationic or preferably nonionic surfactant, or
(IV) free-radical emulsion polymerization of a mixture of monomers (a1), (a2) and at least one basic nitrogen-containing monomer in the presence of a cationic or preferably nonionic surfactant, followed by the protonation of the nitrogen-containing monomer units with an acid.

10. The dispersion of any one of claims 1 to 9, wherein the at least one organic or inorganic fluoride source (d) is selected from sodium fluoride, potassium fluoride, ammonium fluoride, ammonium bifluoride, sodium monofluorophosphate, potassium monofluorophosphate, ammonium hexafluorosilicate, magnesium hexafluorosilicate, potassium hexafluorosilicate, ammonium hexafluorotitanate, ammonium hexafluoroaluminate, zirconium fluoride, tetra-n-butylammonium dihydrogentrifluoride (TBAF-3), rubidium fluoride, cesium fluoride, potassium bifluoride ($KHF_2$), silver(I) fluoride, tin(II) fluoride, Olaflur, Decafluor or a mixture thereof.

11. The dispersion of any one of claims 1 to 10 which additionally comprises at least one further component selected from humectants, antimicrobial compounds, oxidizing agents, sweeteners, colorants, flavors, pH-regulators, soothing agents, and thickeners.

12. The dispersion of claim 11, comprising

(a) 5 to 41 wt.%, preferably 7 to 35 wt.%, and more preferably 15 to 30 wt.% of at least one copolymer,
(b) 50 to 94 wt.%, preferably 60 to 91 wt.%, and more preferably 65 to 83 wt.% of water,
(c) 0.5 to 6.0 wt.%, preferably 0.6 to 5.5 wt.%, and more preferably 0.7 to 5.0 wt.%. of at least one surfactant,
(d) 0.3 to 3.0 wt.%, preferably 0.5 to 2.5 wt.% and more preferably 1.0 to 2.0 wt.% of at least one inorganic or organic fluoride source (calculated as F⁻),
(e) 0 to 3.0 wt.%, preferably 0.1 to 2.0 wt.% and more preferably 0.2 to 1.5 wt.% of one or more low-molecular weight antimicrobial compounds,
(f) 0 to 30 wt.%, preferably 0 to 25 wt.% and more preferably 0 to 20 wt.% of one or more humectants,
(g) 0 to 16 wt.%, preferably 1 to 16 wt.% and more preferably 3 to 13 wt.% of one ore more oxidizing agents, and
(h) 0.01 to 10 wt.%, preferably 0.1 to 8.0 wt.% and more preferably 0.2 to 6.0 wt.% of at least one additive selected from sweeteners, colorants, flavors, pH-regulators, soothing agents, thickeners or a mixture thereof,

all percentages by weight being based on the total weight of the dispersion.

13. The dispersion of any one of claims 1 to 12 for use in a method for the remineralization of teeth, for the treatment or prophylaxis of caries, initial caries lesions (initial caries), white spots, dental erosion, for hardening damaged teeth or for the restoration of hard tooth tissue.

14. Use of a dispersion according to any one of claims 1 to 12, for the fluoridation of teeth.

15. A method for the therapeutic or non-therapeutic treatment of teeth comprising the following steps:

(i) optionally cleaning the tooth or teeth to remove tartar (calculus) or other deposits,
(ii) optionally drying the tooth or teeth,
(iii) applying a dental varnish according to any one of claims 1 to 12 to the surface of at least one tooth, and
(iv) drying the applied varnish.

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 7 781 498 B2 (MALLARD CREEK POLYMERS INC [US]) 24 August 2010 (2010-08-24)<br>* claims 1-22 *<br>* example 1 *<br>* column 4, line 49 - line 62 *<br>* column 14, line 49 - line 62 *<br>* column 16, line 29 - line 39 *<br>----- | 1-15 | INV.<br>A61K6/20<br>A61K8/81<br>C08F2/28<br>C11D3/48 |
| Y | US 2015/257983 A1 (LENDENMANN URS [CH] ET AL) 17 September 2015 (2015-09-17)<br>* paragraph [0014] *<br>* claims 1-23 *<br>----- | 1-15 | |

|  |
|---|
| TECHNICAL FIELDS SEARCHED (IPC) |
| A61K<br>C08F<br>C11D |

The present search report has been drawn up for all claims

| Place of search | Date of completion of the search | Examiner |
|---|---|---|
| The Hague | 15 November 2024 | Gomes Pinto F., R |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 38 2681

This annex lists the patent family members relating to the patent documents cited in the above-mentioned European search report.
The members are as contained in the European Patent Office EDP file on
The European Patent Office is in no way liable for these particulars which are merely given for the purpose of information.

15-11-2024

| Patent document cited in search report | | Publication date | Patent family member(s) | | Publication date |
|---|---|---|---|---|---|
| US 7781498 | B2 | 24-08-2010 | CA | 2679154 A1 | 24-12-2008 |
| | | | CN | 101743123 A | 16-06-2010 |
| | | | EP | 2134324 A2 | 23-12-2009 |
| | | | KR | 20100014559 A | 10-02-2010 |
| | | | US | 2007149694 A1 | 28-06-2007 |
| | | | WO | 2008156509 A2 | 24-12-2008 |
| US 2015257983 | A1 | 17-09-2015 | EP | 2705825 A1 | 12-03-2014 |
| | | | ES | 2707880 T3 | 05-04-2019 |
| | | | US | 2015257983 A1 | 17-09-2015 |
| | | | WO | 2014037337 A1 | 13-03-2014 |

EPO FORM P0459

For more details about this annex : see Official Journal of the European Patent Office, No. 12/82

# EP 4 670 703 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 10242476 A1
- DE 102008010464 A1
- WO 2010043606 A1
- EP 2705825 A1
- WO 2015084315 A1
- DE 102007040569 A1
- US 20080194753 A1
- CN 110591008 A
- EP 0286009 A2
- CN 110627963 A
- CN 102079693 B
- WO 2013066927 A

**Non-patent literature cited in the description**

- **E. D. BELTRÁN-AGUILAR ; J. W. GOLDSTEIN ; S. A. LOCKWOOD**. *J. Amer. Dent. Ass.*, 2000, vol. 131, 589-596
- **A. BAIK ; N. ALAMOUDI ; A. EL-HOUSSEINY ; A. ALTUWIRQI**. *Dent. J.*, 2021, vol. 9 (64), 1-15

18